# EUROPÄISCHE PATENTSCHRIFT

(11) **EP 0 964 855 B1**
(45) Veröffentlichungstag und Bekanntmachung des Hinweises auf die Patenterteilung: **17.10.2001**
(21) Anmeldenummer: 98901950.0
(22) Anmeldetag: 08.01.1998
(51) Int. Cl.: C07D 239/36, C07D 239/40

(54) **VERFAHREN ZUR HERSTELLUNG VON SUBSTITUIERTEN PYRIMIDINDERIVATEN**
PROCESS FOR PREPARING SUBSTITUTED PYRIMIDINE DERIVATIVES
PROCEDE DE PREPARATION DE DERIVES DE PYRIMIDINE SUBSTITUES

(30) Priorität: 13.01.1997 CH 5597
(43) Veröffentlichungstag der Anmeldung: 22.12.1999
(73) Patentinhaber: Lonza AG, 3930 Visp (CH)
(72) Erfinder: SCHMIDT, Beat, CH-3937 Baltschieder (CH); STUCKY, Gerhard, CH-3902 Brig-Glis (CH)
(74) Vertreter: Ritthaler, Wolfgang, Dr.
(86) Internationale Anmeldenummer: EP9800074
(87) Internationale Veröffentlichungsnummer: WO9830549

(56) Entgegenhaltungen:
- EP-A- 0 336 250
- DE-A- 1 806 867
- J. T. GUPTON: ALDRICHIMICA ACTA, Bd. 19, Nr. 2, 1986, Seiten 43-6, XP002064554
- H. BREDERECK ET AL.: CHEMISCHE BERICHTE, Bd. 98, 1965, Seiten 3883-7, XP002064555 in der Anmeldung erwähnt
- K. R. HUFFMAN ET AL.: JOURNAL OF ORGANIC CHEMISTRY, Bd. 27, 1962, Seiten 551-8, XP002064556 in der Anmeldung erwähnt
- H. GOLD: ANGEWANDTE CHEMIE, Bd. 72, Nr. 24, 1960, Seiten 956-9, XP002064557 in der Anmeldung erwähnt

## Beschreibung

Die Erfindung beinhaltet einen neuen Zugang zu substituierten Pyrimidinderivaten der aligemeinen Formel 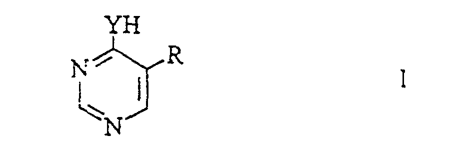 worin Y und R die nachstehend erläuterten Bedeutungen haben. Die genannten Verbindungen haben ein sehr breites Anwendungsspektrum z. B, als Zwischenprodukte für pharmazeutische oder agrochemische Wirkstoffe.

Bekannt ist Verbindungen der allgememen Formel I durch Umsetzung von Trisformarnino-methan mit CH-aciden Carbonsäureamiden zu erhalten (Bredereck et al. Chem. Ber. 1965, 98, 3883-3887). Beispielhaft erwähnt wird die Umsetzung von Trisformamino-methan mit Malonsäureethylesteramid in einer Ausbeute von 11% zum entsprechenden 4-Hydroxypyrimidin-5-carbonsäureethylester (4-Hydroxy-5-ethoxycarbonylpyrimidin).
Ausserdem bekannt ist die Umsetzung von Triazin z. B. mit Malonsäurediethylester in einer Ausbeute von 42% zum 4-Hydroxypyrimidin-5-carbonsaureethylester (Huffmann et al. J.org.Chem. 1962, 27, 551-558).
Diese Synthesen beinhalten den Nachteil, dass sie einerseits relativ schiechte Ausbeuten liefern, andererseits von schwer zugänglichen und relativ teuren Ausgangsverbindungen ausgehen und somit nicht wirtschaftlich sind.
Die Aufgabe der Erfindung bestand folglich darin einen neuen wirtschaftlichen Zugang zu den Verbindungen der allgemeinen Formel I zu entwickeln, der die genannten Nachteile nicht einschliesst.
Die Aufgabe konnte gelöst werden mit dem erfindungsgemässen Verfahren nach Anspruch 1 oder 5.

In der allgemeinen Formel I hat Y die Bedeutung von einem Sauerstoffatom oder einem Schwefelatom.
R steht für Cyano oder für eine Gruppe 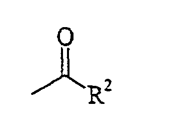 worin R² gegebenenfalls substituiertes Alkyl, Alkoxy, Hydroxy, Amino, Alkylamino, Dialkylamino, Phenyl oder Benzyl bedeutet.
Alkyl hat zweckmässig die Bedeutung von C₁₋₆-Alkyl-, wie Methyl, Ethyl, n-Propyl, iso-Propyl, n-Butyl, sec-Butyl, tert-Butyl, Pentyl und seine Isomeren oder Hexyl und seine Isomeren, vorzugsweise von C₁₋₄-Alkyl. In dieser Bedeutung von Alkyl mitumfasst sind die Alkylreste von Alkoxy, Alkylamino oder Dialkylamino. Die genannten Reste können zudem ein- oder mehrfach substituiert sein mit z. B. Alkyl, Alkoxy, Amino oder Halogen. Halogen steht üblicherweise für Fluor, Chlor, Brom oder Jod, bevorzugt für Chlor oder Brom.
Ausgangsprodukt des erfindungsgemässen Verfahrens gemäss der Variante nach Anspruch 1 ist das [3-(Dimethylamino)-2-azaprop-2-en-1-yliden] dimethylammonium halogenid der allgemeinen Formel 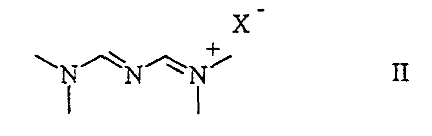 worin X ein Halogenatom bedeutet. Diese Verbindung ist in Form seines Chlorids als "Gold-Reagenz" bekannt. Die Herstellung des Gold-Reagenz erfolgt aaf bekannte Weise durch Umsetzung von Cyanurchlorid mit N,N-Dimethylformamid gemäss H. Gold, Angew. Chem. 1960, 72, 956-959.
Alternativ kann gemäss Anspnlch 5 das Gold-Reagenz bzw. ein anderes Halogenid der allgemeinen Formel II in situ und ohne es zu isolieren für die Folgestufe zur Verfügung gestellt werden.

Bevorzugtes [3-(Dimethylamino)-2-azaprop-2-en-1-yliden] dimethylammonium halogenid der allgemeinen Formel II ist das "Gold-Reagenz" bzw. das [3-(Dimethylamino)-2-azaprop-2-en-1-yliden] dimethylammonium chlorid.

Die erfindungsgemässe Umsetzung mit der Verbindung der allgemeinen Formel II erfolgt mit einer Verbindung der allgemeinen Formel 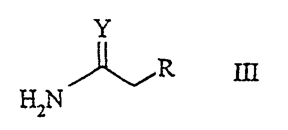 worin Y und R die genannte Bedeutung haben.
Geeignete Verbindungen der allgemeinen Formel III sind die substituierten Acetamide worin Y die Bedeutung hat von Sauerstoff und R die Bedeutung hat von

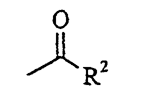

Besonders bevorzugt sind folglich Malonsäurediamid oder die Malonsäure-C₁₋₆-alkylestermonoamide, insbesondere die Malonsäure -C₁₋₄-alkylestermonoamide.

Ebenfalls geeignete Verbindungen der allgemeinen Formel III sind das Cyanacetamid und das 2-Cyan-thioacetamid mit der Bedeutung von R gleich Cyano und Y Sauerstoff bzw. Schwefel.

Das Gold-Reagenz bzw. ein anderes Halogenid der allgemeinen Formel II kann in der Regel stöchiometrisch, bevorzugt aber in einem leichten Überschuss zur Verbindung der allgemeinen Formel III eingesetzt werden.

Die Umsetzung verläuft in Gegenwart einer Base.
Bevorzugt wird ein übliches Alkalialkoholat wie z. B. Na-, oder K-methylat,-ethylat oder tert-butylat eingesetzt.

Zweckmässig verläuft die Reaktion in Gegenwart eines organischen Lösungsmittels wie z. B. Dimethoxyethan, Dioxan, Tetrahydrofuran oder Methanol.
Die Reaktionstemperatur wählt man üblicherweise im Bereich zwischen 20°C und 100°C . Bevorzugt wird bei Rückflusstemperatur gearbeitet.

Nach einer Reaktionszcit von 1 h bis 20 h kann das resultierende Pyrimidin der allgemeinen Formel I auf einfache Weise, in der Regel durch Filtration, dem Reaktionsgemisch entnommen werden.
In dem Fall wo im resultierenden Pyrimidin der allgemeinen Formel I R die Bedeutung einer Esterfunktion hat, kann durch saure oder basische Aufarbeitung auf einfache Weise die entsprechende Pyrimidincarbonsäure erhalten werden.
Mit dem erfindungsgemässen Verfahren lassen sich die substituierten Pyrimidinderivate der allgemeinen Formel I in guten Ausbeuten von über 80% gewinnen.

### Beispiele:

### Beispiel 1

### Verfahren zur Herstellung von [3-(Dimethylamino)-2-azaprop-2-en-1-yliden] dimethylammonium chlorid (GoId-Reagenz)

Cyanurchlorid (46,67 g, 0,252 mol) wurde mit N,N-Dimethyiforrnamid (121,33 g, 1,66 mol) in 250 ml tert. Butylmethylether während 30 bis 40 min erhitzt. Danach trat eine exotherme Reaktion auf, und während 1 bis 2 Stunden wurde CO₂-Gas frei. Das Reaktionsgemisch wurde über Nacht gekocht. Nach Abkühlen des Reaktionsgemisches liess sich das Titelprodukt durch rasche Filtration unter N₂-Atmosphäre und Waschen mit tert. Butylmethylether isolieren. Nach Trocknen im Exsikkator isolierte man das Titelprodukt in einer Ausbeute von 112,15 g (90,7%) in Form eines leicht gelben Feststoffes.
Fp = 103 °C
- ¹H NMR (DMSO_{d6}, 400 MHz) δ =: 3,2 (s,6H);
3,27 (s, 6H);
8,62 (s, 2H).

### Beispiel 2a

### Verfahren zur Herstellung von 4-Hydroxypyrimidin-5-carbonsäuremethylester

Zum vorgelegten Natriummethylat (279,4 g, 1,5 mol, 29% in Methanol) gab man Oxalsäuremethylester (0,2 eq.) und Malonsäuremonomethylestermonoamid (58,55 g, 0,50 mol). Man fügte 350 ml Dimethoxyethan zu. Dann gab man das feste, gemäss Beispiel 1 hergestellte Goldreagenz (89,35 g, 0,55 mol) spatelweise zu. Man liess die Suspension während 18 Stunden bei Raumtemperatur rühren. Die gelbe Suspension wurde auf eine wässrige, eiskalte Salzsäureiösung (2 eq. HCl) gegossen. Nach Filtration bei pH 9-10 wurde das feuchte Natriumsalz des Titelproduktes in HCl-gesättigtem Methanol aufgenommen, 15 min gerührt, filtriert und getrocknet.
Die Ausbeute des Titelproduktes in Form seines Hydrochlorids betrug 52,3 g (55%).
- ¹H NMR (DMSO_{d6}, 400 MHz) δ =: 3,8 (s, 3H); 5,7 - 6,4 (breit, 1H);
8,4 (s, 1H);
8,5 (s, 1H).

### Beispiel 2b

### Verfahren zur Herstellung von 4-Hydroxypyrimidin-5-carbonsäure

Das gemäss Beispiel 2a) erhaltene feuchte Nairiumsalz des 4-Hydroxypyrimidin-5-carbonsäuremethylesters wurde in Wasser/Tetrahydrofuran = 1 : 1 aufgenommen und 1,0 eq LiOH.H₂O zugefügt. Nach Hydrolyse des Esters über 3,5 Stunden bei 0 °C und 2 Stunden bei Raumtemperatur wurde das Tetrahydrofuran weitgehend eingedampft und bei 0 °C mit aq. HCl-Lösung ein pH von 3 eingestellt. Nach ca. 45 Minuten bei 0 °C wurde das Titelprodukt abfiltriert. Nach Trocknen wurde die Säure in einer Ausbeute von 3,55 g (78%) erhalten.
- ¹H NMR (DMSO_{d6}, 400 MHz) δ =: 8,6 (s, 1H);
8,7 (s, 1H);
11,8 - 14,7 (breit, 2H).

### Beispiel 3

### Verfahren zur Herstellung von 4-Hydroxypyrimidin-5-carbonsäureamid

Zu einer Natriummethylat Lösung (55,88 g, 0,30 mol, 29% in Methanol) fügte man bei Raumtemperatur Malonsäurediamid (10,75 g, 0,10 mol) zu und verdünnte mit Tetrahydrofuran. Man gab bei Raumtemperatur das gemäss Beispiel 1 hergestellte Goldreagenz (18,0 g, 0,11 mol) zu und leitete während 24 Stunden Argon durch, um das freiwerdende Dimelylamin auszutreiben. Der pH-Wert wurde dann mit einer wässrigen Salzsäurelösung auf 7,4 eingestellt und das Tetrahydrofuran am Rotationsverdampfer weitgehend entfernt. Die erhaltene Suspension wurde mit aq. HCl auf pH = 5,5 gestellt und auf Rückflusstemperatur erhitzt. Man fügte nochmals Wasser zu, um alles in Lösung zu bringen, dann gab man Aktivkohle zu liess weitere 15 min unter Rückfluss rühren. Nach langsamem Abkühlen auf Raumtemperatur fielen leicht gelbe Kristalle aus, die nach Filtration unter Vakuum bei 40°C getrocknet wurden. Man erhielt das Titelprodukt in einer Ausbeute von 5,58 g (80 %).
- ¹H NMR (DMSO_{d6}, 400 MHz) δ =: 7,7 (s, breit, 1H);
8,4 (s, 1H);
8,5 - 8,6 (s, breit, 1H);
8,6 (s, 1H).

### Beispiel 4

### Verfahren zur Herstellung von 5-Cyanpyrimidinol

Zum vorgelegten Natriummethylat (54,02 g, 0,30 mol, 29% in Methanol) gab man Cyanacetamid (8,58 g, 0,10 mol) Man fügte 150 ml Dimexhoxyethan zu. Dann gab man das feste, gemäss Beispiel 1 hergestellte Goldreagenz (18,08 g, 0,11 mol) spatelweise zu. Man liess die Suspension über Nacht bei Raumtemeperatur rühren. Die gelbe Suspension wurde auf eine wässrige, eiskalte Salzsäurelösung (2 eq. HCl) gegossen und auf pH 5,5 gestellt. Nach Abdampfen der org. Lösungsmittel fiel das Produkt aus der Wasserphase aus. Durch Digerieren in Wasser und anschliessende Filtration konnte man das Titelprodukt in einer Ausbeute von: 9,14 g (75%) in Form eines gelben Feststoffs erhalten.
- ¹H NMR (DMSO_{d6}, 400 MHz) δ =: 8,2 (s, 1H);
8,3 (s, 1H).

### Beispiel 5

### Verfahren zur Herstellung von 5-Cyan-4-pyrimidin-thiol

Zum vorgelegten Natriummethylat (24,3 g, 0,135 mol, 29% in Methanol) fügte man bei Raumtemperatur 2-Cyan-thioacetamid (4,5 g, 0,045 mol). Man fügte 100 ml Dimethoxyethan zu. Anschliessend gab man das gemäss Beispiel 1 hergestellte Goldreagenz ( 8,1g, 0,050 mol) zu und liess das Reaktionsgemisch ca. 5 Stunden bei Raumtemperatur rühren. Man erhitzte kurz auf 50 °C und liess über Nacht bei Raumtemperatur stehen. Das Dimethoxyethan wurde am Rotationsverdampfer abgezogen, 20 ml Wasser wurden zugefügt, dann wurde das Reaktionsgemisch im Eisbad abgekühlt. Mit 30 ml 2N aq HCl-Lösung wurde der pH auf 5,5 eingestellt. Nach erneutem Abkühlen wurde der ausgefallene Feststoff abfiltriert, mit wenig kaltem Wasser nachgewaschen und bei 40 °C im Vakuumtrockenschrank getrocknet. Man erhielt so das Titelprodukt in einer Ausbeute von 5,57 g (90,4%) in Form eines orangen Feststoffes.
- ¹H NMR (DMSO_{d6}, 400 MHz) δ =: 8,42 (s, 1H);
8,48 (s, 1H).

## Patentansprüche

1. Verfahren zur Herstellung von substituierten Pyrimidinderivaten der allgemeinen Formel 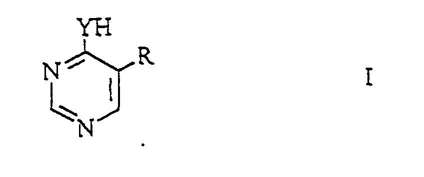 worin Y ein Sauerstoffatom oder ein Schwefelatom bedeutet und R Cyano oder eine Gruppe 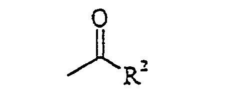 bedeutet, worin R² für gegebenenfalls mit Alkyl, Alkoxy, Amino und/oder Halogen substituiertes Alkyl, Alkoxy, Hydroxy, Amino, Alkylamino, Dialkylamino, Phenyl oder Benzyl steht, **dadurch gekennzeichnet, dass** man ein [3-(Dimethylamino)-2-azaprop-2-en-1-yliden] dimethylammoniumhalogenid der allgemeinen Formel 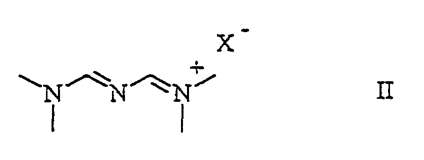 worin X ein Halogenatom bedeutet, mit einer Verbindung der allgemeinen Formel 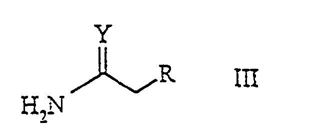 worin Y und R die genannte Bedeutung haben, in Gegenwart einer Base zur Umsetzung bringt

2. Verfahren nach Anspruch 1, **dadurch gekennzeichnet, dass** als Verbindung der allgemeinen Formel II [3-(Dimethylamino)-2-azaprop-2-en-1-yliden] dimethylammonium chlorid verwendet wird.

3. Verfahren nach Anspruch 1 oder 2, **dadurch gekennzeichnet, dass** als Base ein Alkalialkoholat eingesetzt wird.

4. Verfahren nach einem der Ansprüche 1 bis 3, **dadurch gekennzeichnet, dass** die Umsetzung in Gegenwart eines Lösungsmittels bei einer Temperatur zwischen 20°C und 100°C durchgeführt wird.

5. Verfahren zur Herstellung von substituierten Pyrimidinderivaten der allgemeinen Formel 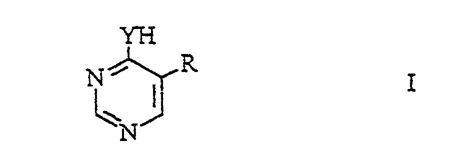 worin Y ein Sauerstoffatom oder ein Schwefelatom bedeutet und R Cyano oder eine Gruppe 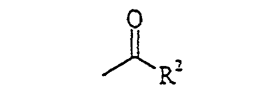 bedeutet, worin R² für gegebenenfalls mit Alkyl, Alkoxy, Amino und/oder Halogen substituiertes Alkyl, Alkoxy, Hydroxy, Amino, Alkylamino, Dialkylamino, Phenyl oder Benzyl steht, **dadurch gekennzeichnet, dass** in der ersten Stufe ein Cyanurhalogenid mit N,N-Dimethylformamid in ein [3-(Dimethylamino)-2-azaprop-2-en-1-yliden] dimethylammoniumhalogenid der allgemeinen Formel 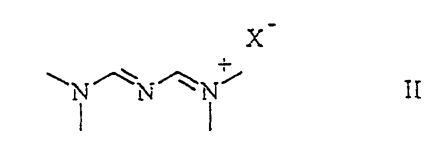 worin X ein Halogenatom bedeutet, uberführt wird und darauf in der zweiten Stufe mit einer Verbindung der allgemeinen Formel 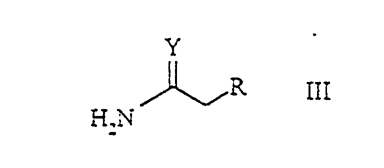 worin Y und R die genannte Bedeutung haben, in Gegenwart einer Base zur Umsetzung gebracht wird.

6. Verfahren nach Anspruch 5, **dadurch gekennzeichnet, dass** als Verbindung der allgemeinen Formel II [3-(Dimethylamino)-2-azaprop-2-en-1-yliden] dimethylammoniumchlorid verwendet wird.

7. Verfahren nach Anspruch 5, **dadurch gekennzeichnet, dass** die Umsetzung in der ersten Stufe mit Cyanurchlorid durchgeführt wird.

8. Verfahren nach einem der Ansprüche 5 bis 7, **dadurch gekennzeichnet, dass** als Base ein Alkalialkoholat eingesetzt wird.

9. Verfahren nach einem der Ansprüche 5 bis 8, **dadurch gekennzeichnet, dass** die Umsetzung in der zweiten Stufe in Gegenwart eines Lösungsmittels bei einer Temperatur zwischen 20°C und 100°C durchgeführt wird.

10. Verfahren nach einem der Ansprüche 5 bis 9 **dadurch gekennzeichnet, dass** das aus der ersten Stufe resultierende [3-(Dimethylamino)-2-azaprop-2-en-1-yliden] dimethylammoniumhalogenid nicht isoliert wird.

## Claims

1. Process for the production of substituted pyrimidine derivatives of the general formula 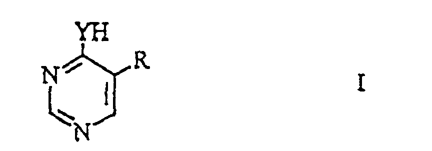 wherein Y signifies an oxygen atom or a sulfur atom and R denotes cyano or a 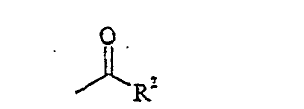 group, wherein R² denotes optionally alkyl-, alkoxy-, amino- and/or halogen-substituted alkyl, alkoxy, hydroxy, amino, alkylamino, dialkylamino, phenyl or benzyl, **characterised in that** a [3-(dimethylamino)-2-azaprop-2-en-1-ylidene] dimethylammonium halide of the general formula 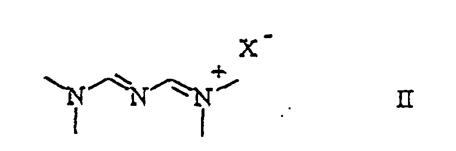 wherein X signifies a halogen atom, is reacted with a compound of the general formula 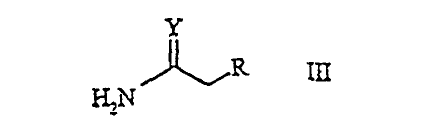 wherein Y and R have the above-mentioned meaning, in the presence of a base.

2. Process according to claim 1, **characterised in that** [3-(dimethylamino)-2-azaprop-2-en-1-ylidene] dimethylammonium chloride is used as the compound of general formula II.

3. Process according to claim 1 or 2, **characterised in that** an alkali alkoxide is used as the base.

4. Process according to one of claims 1 to 3, **characterised in that** the reaction is performed in the presence of a solvent at a temperature of between 20°C and 100°C.

5. Process for the production of substituted pyrimidine derivatives of the general formula 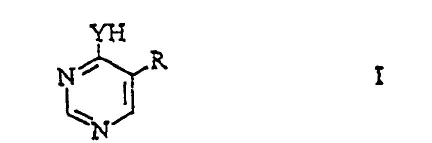 wherein Y signifies an oxygen atom or a sulfur atom and R denotes cyano or a 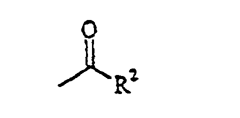 group, wherein R² denotes optionally alkyl-, alkoxy-, amino- and/or halogen-substituted alkyl, alkoxy, hydroxy, amino, alkylamino, dialkylamino, phenyl or benzyl, **characterised in that** in the first step a cyanuric halide is converted with N,N-dimethyl formamide into a [3-(dimethylamino)-2-azaprop-2-en-1-ylidene]dimethylammonium halide of the general formula 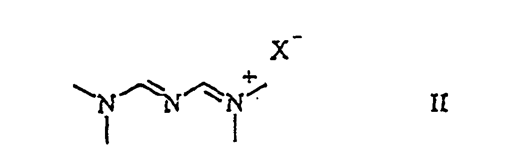 wherein X signifies a halogen atom, and then in the second step is reacted with a compound of the general formula 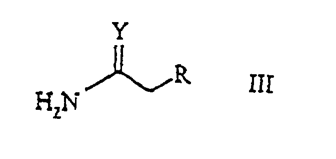 wherein Y and R have the above-mentioned meaning, in the presence of a base.

6. Process according to claim 5, **characterised in that** [3-(dimethylamino)-2-azaprop-2-en-1-ylidene] dimethylammonium chloride is used as the compound of general formula II.

7. Process according to claim 5, **characterised in that** the reaction in the first step is performed with cyanuric chloride.

8. Process according to one of claims 5 to 7, **characterised in that** an alkali alkoxide is used as the base.

9. Process according to one of claims 5 to 8, **characterised in that** the reaction in the second step is performed in the presence of a solvent at a temperature of between 20°C and 100°C.

10. Process according to one of claims 5 to 9, **characterised in that** the [3-(dimethylamino)-2-azaprop-2-en-1-ylidene]dimethylammonium halide resulting from the first step is not isolated.

## Revendications

1. Procédé pour la préparation de dérivés de pyrimidine substitués de la formule générale 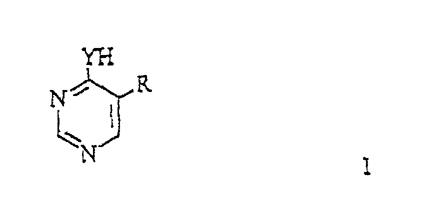 dans laquelle Y signifie un atome d'oxygène ou un atome de soufre et R signifie cyano ou un groupe 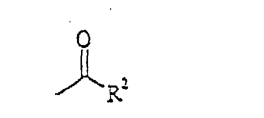 dans laquelle R² désigne éventuellement benzyle ou phényle, dialkylamino, alkylamino, amino, hydroxy, alcoxy, alkyle éventuellement substitué par alkyle, alcoxy, amino et/ou halogène,
**caractérisé en ce que** l'on met en réaction en présence d'une base un halogénure de 3-(diméthylamino)-2-azaprop-2-en-I-yliden diméthylammonium de la formule générale 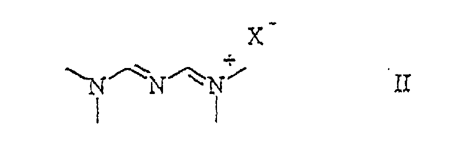 dans laquelle X signifie un atome halogène, avec un composé de la formule générale 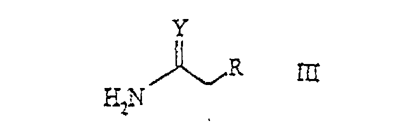 dans laquelle Y et R ont la signification citée.

2. Procédé selon la revendication 1, **caractérisé en ce que** comme composé de la formule générale II on utilise le chlorure de 3-(diméthylamino)-2-azaprop-2-en-1-yliden diméthylammonium.

3. Procédé selon la revendication 1 ou 3, **caractérisé en ce qu'**en tant que base on utilise un alccolat alcalin.

4. Procédé selon les revendications 1 à 3, **caractérisé en ce que** la réaction est conduite en présence d'un solvant à une température entre 20 et 100°C.

5. Procédé pour la préparation de dérivés de pyrimidine substitués de la formule générale 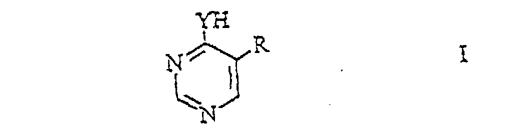 dans laquelle Y signifie un atome d'oxygène ou un atome de soufre et R signifie cyano ou un groupe 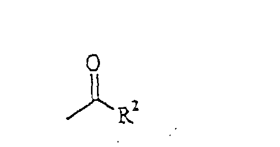 dans laquelle R² désigne éventuellement phényle ou benzyle, dialkylamino, alkylamino, amino, hydroxy, alcoxy, alkyle substitué par alkyle, alcoxy, amino et/ou halogène,
**caractérisé en ce que** dans la première étape un halogénure de cyanure avec N,N-diméthylformamide est transformé en un diméthylammonium halogénure de 3-(diméthylamino)-2-azaprop-2-en-1-yliden de la formule générale 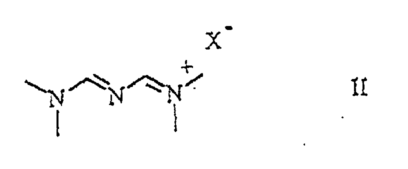 dans laquelle X signifie un atome halogène et ensuite dans une deuxième étape en présence d'une base avec un composé de la formule générale 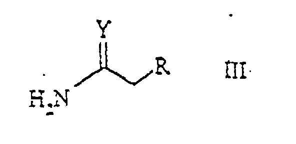 dans laquelle Y et R ont la signification citée.

6. Procédé selon la revendication 5, **caractérisé en ce que** comme composé de la formule générale II on utilise le chlorure de 3-(diméthylamino)-2-azaprop-2-en-1-yliden diméthylammonium.

7. Procédé selon la revendication 5, **caractérisé en ce que** dans la première étape la réaction est conduite avec le chlorure de cyanure.

8. Procédé selon l'une des revendications 5 à 7, **caractérisé en ce que** comme base on utilise un alcoolat alcalin.

9. Procédé selon l'une des revendications 5 à 8, **caractérisé en ce que** dans la deuxième étape la réaction est conduite en présence d'un solvant à une température entre 20 et 10°C.

10. Procédé selon l'une des revendications 5 à 9, **caractérisé en ce que** l'halogénure de 3-(diméthylamino)-2-azaprop-2-en-1-yliden diméthylammonium résultant n'est pas isolé.
